# EUROPEAN PATENT APPLICATION

(11) **EP 4 385 543 A2**
(43) Date of publication of application: **19.06.2024**
(21) Application number: 22856242.7
(22) Date of filing: 11.08.2022
(51) Int. Cl.: A61M 5/315, A61M 5/155, A61M 5/142

(54) **MEDICINAL LIQUID INJECTION APPARATUS**

(30) Priority: 12.08.2021 KR 20210106471
(71) Applicant: Kim, Yong Hyun, Goyang-si, Gyeonggi-do 10483 (KR)
(72) Inventor: Kim, Yong Hyun, Goyang-si, Gyeonggi-do 10483 (KR)
(74) Representative: Santarelli
(86) International application number: PCT/KR2022/012008
(87) International publication number: WO 2023/018248

(57) **Abstract**

A medicinal liquid injection apparatus according to an embodiment of the present disclosure includes: a chamber configured to store a medicinal liquid in the chamber; a piston configured to slide in the chamber in a predetermined pressurizing direction to pressurize the medicinal liquid stored in the chamber; and a pressurizing actuator configured to increase pressure of gas in a pressurizing space formed in a direction opposite to the pressurizing direction of the piston to press the piston, wherein the pressurizing actuator includes: a receptacle configured to accommodate a liquid substance and a solid substance that generates gas upon coming into contact with the liquid substance; a partition disposed between the liquid substance and the solid substance to isolate the liquid substance and the solid substance from each other; a rotary part rotatably coupled to the receptacle; and a pressing part configured to be pressed and moved in a first direction approaching the receptacle as the rotary part rotates, and press the receptacle by moving in the first direction to release an isolation by the partition.

## Description

### TECHNICAL FIELD

The present disclosure relates to a medicinal liquid injection apparatus.

### BACKGROUND

There is known a medicinal liquid injection apparatus for injecting a medicinal liquid into a patient in order to supply the medicinal liquid to the patient. By using the medicinal liquid injection apparatus, a medicinal liquid within a predetermined storage space flows into the patient's body through a passage connected to the patient (e.g., the inner spaces of a tube and an injection needle).

An exemplary medicinal liquid injection apparatus includes a chamber capable of storing a medicinal liquid therein, and a piston configured to pressurize the medicinal liquid stored in the chamber. The exemplary medicinal liquid may be a gas-generating medicinal liquid injection apparatus that uses gas pressure to press and move a piston. In the gas-generating medicinal liquid injection apparatus, an injection piston within a cylinder is stably and slowly moved forward by the generated high gas pressure, and the forward movement of the injection piston allows the medicinal liquid filled in the medicinal liquid storage space in the chamber to be stably and continuously injected into a patient at a constant flow rate.

The gas-generating medicinal liquid injection apparatus generates gas (e.g., carbon dioxide) by reacting a liquid substance such as citric acid with a solid substance such as sodium bicarbonate, and uses the pressure of the generated gas to advance the piston and inject a medicinal liquid.

In the gas-generating medicinal liquid injection apparatus, the liquid substance and the solid substance for generating gas are isolated from each other by a partition, and the generation of gas is prevented before use. When a user needs to use the medicinal liquid injection apparatus, the liquid substance and solid substance can be brought into contact with each other by releasing an isolation by the partition.

A gas-generating medicinal liquid injection apparatus may be used, in which a user uses his or her hands to press a specific portion of a receptacle where a solid substance and a liquid substance are accommodated in order to release the partition isolating the liquid substance and the solid substance from each other.

### SUMMARY

Embodiments of the present disclosure provide a medicinal liquid injection apparatus having a rotary release structure.

The exemplary medicinal liquid injection apparatus, which is configured to release the partition by direct pressure from a user, has a problem in that it is difficult to use the medicinal liquid injection apparatus when the user is in a situation where it is not appropriate to apply a certain amount of force (for example, when the user is a sick patient who lacks strength). Embodiments of the present disclosure solve this problem of the prior art.

The exemplary medicinal liquid injection apparatus, in which the partition is released by direct pressure from a user, has a problem in that the user has to exert more than a certain amount of force, making it inconvenient to use, and even though the partition was not released because the user failed to apply more than a certain amount of force, the user may mistakenly believe that the partition was released. Embodiments of the present disclosure provide a structure that can release an isolation by the partition more easily and reliably, and solve the above-described problems.

An aspect of the present disclosure provides embodiments of a medicinal liquid injection apparatus. A medicinal liquid injection apparatus according to a representative embodiment includes: a chamber configured to store a medicinal liquid in the chamber; a piston configured to slide in the chamber in a predetermined pressurizing direction to pressurize the medicinal liquid stored in the chamber; and a pressurizing actuator configured to increase pressure of gas in a pressurizing space formed in a direction opposite to the pressurizing direction of the piston to press the piston. The pressurizing actuator includes: a receptacle configured to accommodate a liquid substance and a solid substance that generates gas upon coming into contact with the liquid substance; a partition disposed between the liquid substance and the solid substance to isolate the liquid substance and the solid substance from each other; a rotary part rotatably coupled to the receptacle; and a pressing part configured to be pressed and moved in a first direction approaching the receptacle as the rotary part rotates, and press the receptacle by moving in the first direction to release an isolation by the partition.

In an embodiment, the rotation axis of rotary motion of the rotary part with respect to the receptacle may be parallel to the first direction.

In an embodiment, the rotary part may include at least one guide portion having a first inclined surface, a height of which in the first direction is lowered along a first rotation direction of the rotary part, and when the rotary part rotates in the first rotation direction, the pressing part may be configured to move in the first direction by sliding along the first inclined surface of the guide portion.

In an embodiment, the at least one guide portion may further include a second inclined surface, a height of which in the first direction is lowered along a second rotation direction opposite to the first rotation direction of the rotary part, and when the rotary part rotates in the second rotation direction, the pressing part may be configured to move in the first direction by sliding along the second inclined surface of the guide portion.

In an embodiment, the pressing part may include: a plate portion disposed inside the rotary part and configured to press the receptacle; and at least one protrusion configured to be in contact with the guide portion and extending outward from the plate portion.

In an embodiment, the at least one guide portion may include a plurality of guide portions arranged along the rotation direction of the rotary part, and the at least one protrusion may include a plurality of protrusions each arranged along a circumference of the plate portion to be in contact with each of the plurality of guide portions, respectively.

In an embodiment, the receptacle may include an insertion groove into which the protrusion is inserted, and the insertion groove may extend in the first direction to guide the movement of the protrusion in the first direction and to limit the movement of the protrusion in a lateral direction transverse to the first direction.

In an embodiment, the pressing part may include a convex portion protruding convexly in the first direction and configured to press a predetermined portion of the receptacle.

In an embodiment, the receptacle may include: a liquid accommodation portion configured to accommodate the liquid substance; and a solid accommodation portion having an opening open in a direction facing the liquid accommodation portion and configured to accommodate the solid substance, wherein the predetermined portion is located in the solid accommodation portion. The partition may isolate the liquid substance and the solid substance from each other by closing the opening at a closed position.

According to the embodiments of the present disclosure, the user can more easily and reliably release an isolation by the partition by rotating the rotary part.

According to the embodiments of the present disclosure, even if a user is a sick patient with insufficient strength, the user can easily use the medicinal liquid injection apparatus by releasing the isolation by the partition without difficulty.

According to an embodiment of the present disclosure, the isolation by the partition can be released regardless of the rotation direction of the rotary part. Accordingly, user convenience when using the medicinal liquid injection apparatus can be improved, and the medicinal liquid injection apparatus can be used more efficiently.

### BRIEF DESCRIPTION OF THE DRAWINGS

FIG. 1 is a perspective view of a medicinal liquid injection apparatus according to an embodiment.
FIG. 2 is a cross-sectional view of the medicinal liquid injection apparatus taken along line A-A' in FIG. 1.
FIG. 3 is a cross-sectional perspective view of the medicinal liquid injection apparatus sectioned along line A-A' in FIG. 1.
FIG. 4 is an exploded perspective view of the medicinal liquid injection apparatus illustrated in FIG. 1.
FIG. 5 is an enlarged view of a medicinal liquid inflow part in the medicinal liquid injection apparatus illustrated in FIG. 4.
FIG. 6 is a cross-sectional perspective view illustrating the medicinal liquid inflow part of FIG. 5 partially sectioned in a state in which a cap and a base are separated
FIG. 7 is an enlarged view illustrating a portion including the medicinal liquid inflow part in the medicinal liquid injection apparatus illustrated in FIG. 3.
FIG. 8 is a view illustrating a portion including the medicinal liquid inflow part in FIG. 7 when the packing is pressed from the outside and elastically deformed.
FIG. 9 is an elevation view of the medicinal liquid inflow portion and the hydrophobic filter illustrated in FIG. 2 when viewed in the directions of arrows B and B'.
FIG. 10 is a view in which the hydrophobic filter in FIG. 9 is omitted.
FIG. 11 is an exploded perspective view of the hydrophobic filter illustrated in FIG. 9.
FIG. 12 is a cross-sectional view of the hydrophobic filter illustrated in FIG. 11 taken in a lateral direction perpendicular to the stacking direction of first and second hydrophobic filter layers.
FIG. 13 is an enlarged view of the pressurizing actuator in the medicinal liquid injection apparatus illustrated in FIG. 2.
FIG. 14 is a cross-sectional view of the pressurizing actuator taken along line C-C' in FIG. 13.
FIG. 15 is a view in which a plurality of pressurizing filters in FIG. 14 are omitted.
FIG. 16 is a perspective view of the pressing-part filter illustrated in FIG. 14.
FIG. 17 is a projection view of an aspect of the pressing actuator illustrated in FIG. 13.
FIG. 18 is a cross-sectional view of the pressurizing actuator taken along line D-D' in FIG. 17.
FIG. 19 is a projection view of another aspect of the pressing actuator illustrated in FIG. 17.
FIG. 20 is a cross-sectional view of the pressurizing actuator taken along line E-E' in FIG. 19.

### DETAILED DESCRIPTION

Embodiments of the present disclosure are exemplified for the purpose of explaining the technical idea of the present disclosure. The scope of the present disclosure is not limited to the embodiments presented below or the detailed descriptions of such embodiments.

In the present disclosure, "embodiments" are arbitrary divisions for easily explaining the technical idea of the present disclosure, and individual embodiments do not need to be mutually exclusive. For example, configurations disclosed in one embodiment may be applied and implemented in other embodiments, and may be applied and implemented with changes without departing from the scope of the present disclosure.

Technical and scientific terms used in the present disclosure have the meaning generally understood by those of ordinary skill in the art to which the present disclosure pertains, unless otherwise defined. Terms used in the present disclosure are chosen for the purpose of more clearly explaining the present disclosure and are not chosen to limit the scope of rights according to the present disclosure.

As used in the present disclosure, expressions such as "comprising", "including", "having", and the like are to be understood as open-ended terms having the possibility of encompassing other embodiments, unless otherwise mentioned in the phrase or sentence containing such expressions.

As used in the present disclosure, singular expressions may encompass plural meanings unless otherwise stated, and this also applies to the singular expressions recited in the claims.

As used in the present disclosure, expressions such as "first" and "second" used in the present disclosure are used to distinguish a plurality of elements from each other, and are not intended to limit the order or importance of the corresponding elements.

As used in the present disclosure, direction indicators such as "upward" and "upper" refer to the direction in which the chamber 100 is disposed with respect to the pressurizing actuator 300, and direction indicators "downward" and "down" refer to the opposite direction to the upward direction. In addition, in the present disclosure, "horizontal direction" means any one of the directions crossing "up and down direction". This is merely a standard for explaining the present disclosure so that the present disclosure can be clearly understood, and of course, the terms "upward" and "downward" may be defined differently depending on where the standard is placed.

Hereinafter, embodiments of the present disclosure will be described with reference to the accompanying drawings. In the accompanying drawings, identical or corresponding components are assigned the same reference numerals. In addition, in the following description of embodiments, redundant descriptions of the identical or corresponding components may be omitted. However, even if a description of a component is omitted, such a component is not intended to be excluded from an embodiment.

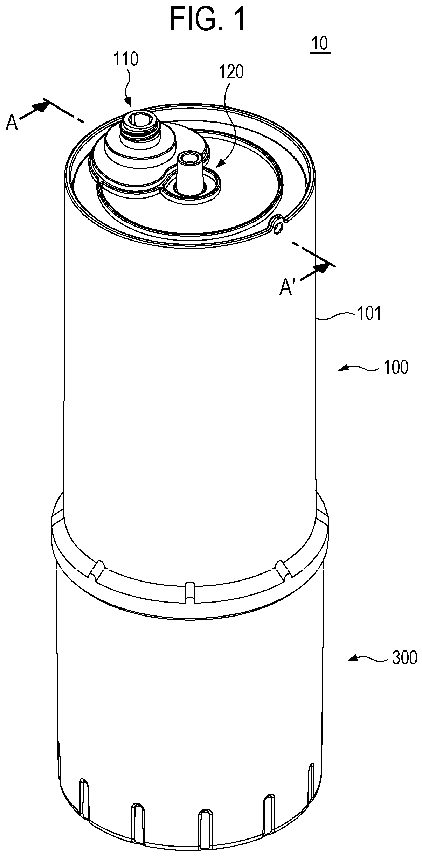

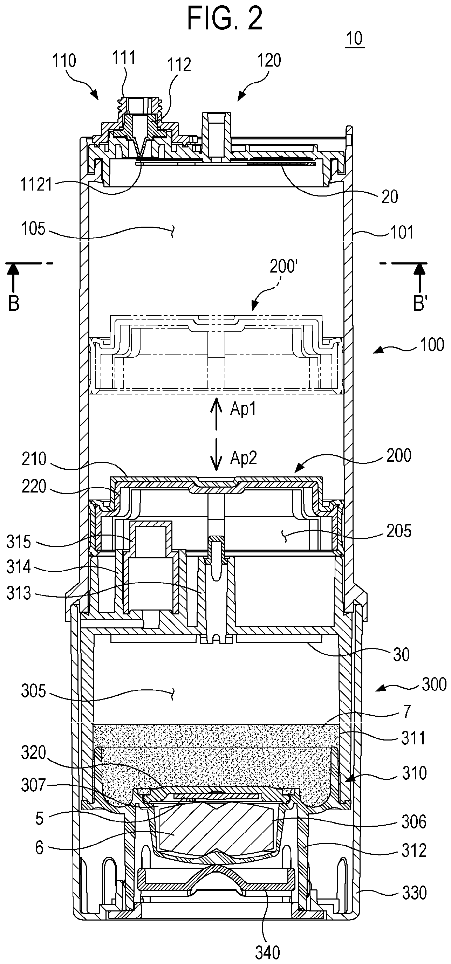

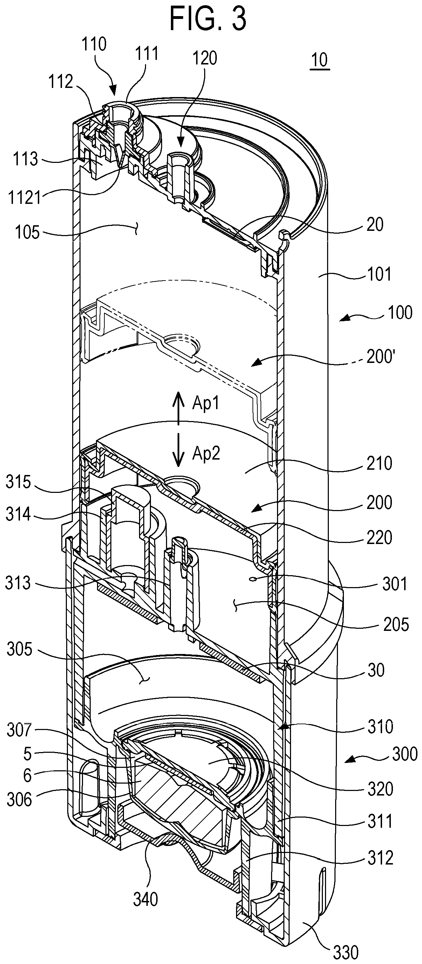

FIG. 1 is a perspective view of a medicinal liquid injection apparatus 10 according to an embodiment, FIG. 2 is a cross-sectional view of the medicinal liquid injection apparatus taken along line A-A' in FIG. 1, and FIG. 3 is a cross-sectional perspective view of the medicinal liquid injection apparatus sectioned along line A-A' in FIG. 1.

The medicinal liquid injection apparatus 10 may include a chamber 100, a piston 200 and a pressurizing actuator 300.

Referring to FIGS. 1 to 3, the chamber 100 may include a chamber housing 101. The chamber housing 101 may be substantially cylindrical. A lubricant may be applied to the inner surface of the chamber housing 101. The chamber 100 may include a medicinal liquid inflow part 110 configured to allow a medicinal liquid to flow into the chamber 100. The chamber 100 may include a medicinal liquid injection part 120 configured to be capable of discharging the stored medicinal liquid. The medicinal liquid discharged through the medicinal liquid injection part 120 may be injected into a patient through a connection tube (not illustrated), an injection needle (not illustrated), and/or a catheter (not illustrated). In the present embodiment, the medicinal liquid inflow part 110 and the medicinal liquid injection part 120 are illustrated as separate components, but this is merely an embodiment, and the medicinal liquid inflow part 110 and the medicinal liquid injection part 120 may be configured as the same integrated component. In another example (not illustrated), the chamber 100 may include a single component configured to allow the inflow and discharge of a medicinal liquid (that is, a component that performs both the functions of the medicinal liquid inflow part and the medicinal liquid injection part).

A medicinal liquid storage space 105 is formed inside the chamber 100. The medicinal liquid storage space 105 may be configured to store the medicinal liquid introduced through the medicinal liquid inflow part 110. The medicinal liquid stored in the medicinal liquid storage space 105 may be discharged through the medicinal liquid injection part 120.

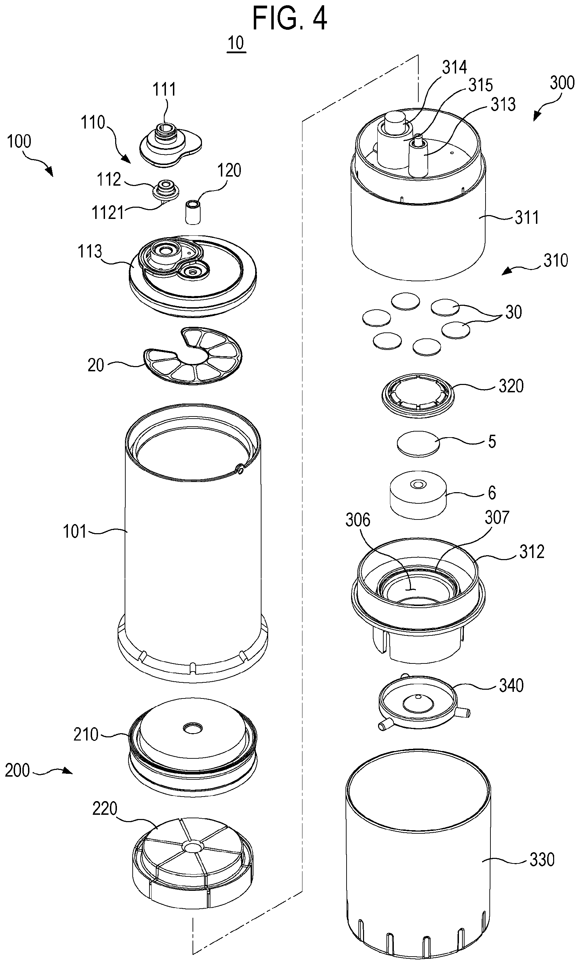

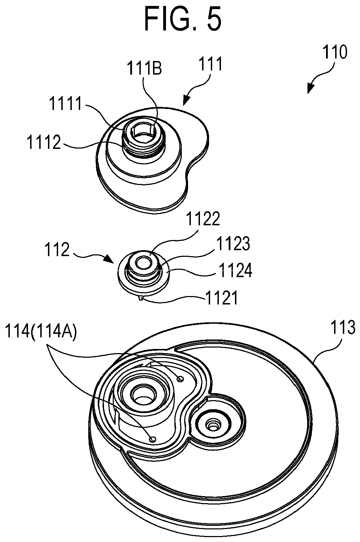

FIG. 4 is an exploded perspective view of the medicinal liquid injection apparatus 10 illustrated in FIG. 1. Referring to FIG. 4 together, the medicinal liquid inflow part 110 may include a cap 111. The cap 111 may be a portion that comes into contact with a medicinal liquid inflow tool (e.g., a syringe) for introducing a medicinal liquid into the interior of the chamber 100. The medicinal liquid inflow part 110 may include a packing 112 made of a flexible material and having a medicinal liquid inflow hole 1121 formed in the center. The flexible material used in the present disclosure is a material that is elastically deformable when pressed from the outside, and may be, for example, rubber, polymer, or the like. When the packing 112 made of the flexible material is pressed from the outside, the packing 112 can be deformed to a predetermined size by being bent or compressed. The medicinal liquid inflow hole 1121 may be configured to allow a medicinal liquid to flow into the chamber 100 from the outside. The medicinal liquid inflow part 110 may include a base 113 coupled to the cap 111 with the packing 112 therebetween. The cap 111, the packing 112, and the base 113 may be arranged from top to bottom in that order. The cap 111, the packing 112, and the base 113 may be coupled to each other to maintain an airtight state. In relation to the cap 111, packing 112, and base 113, which are components of the medicinal liquid inflow part 110, an openable/closable gas passage will be described later with reference to FIGS. 5 to 8.

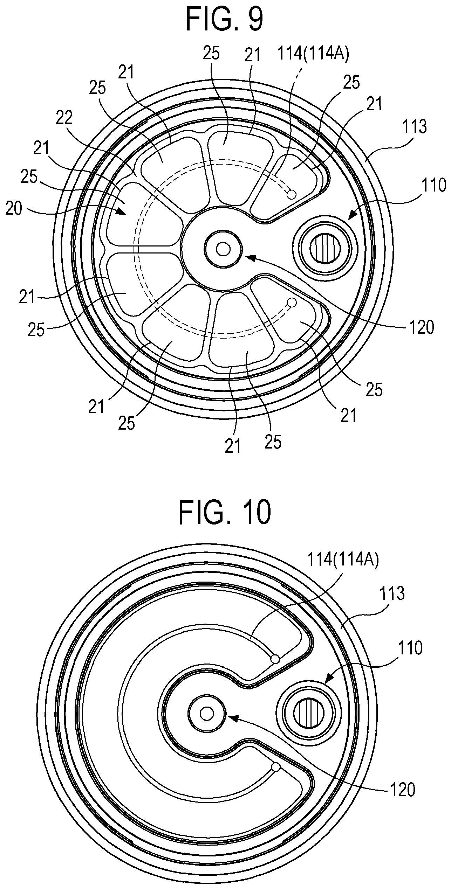

The medicinal liquid injection apparatus 10 may include a hydrophobic filter 20 that is impermeable to liquid and permeable to gas. The hydrophobic filter 20 may be disposed in a predetermined passage to allow gas to pass through the predetermined passage and to prevent liquid from passing through the predetermined passage. The hydrophobic filter 20 will be described later with reference to FIGS. 9 to 12.

Referring to FIGS. 2 and 3 again, the piston 200 may be configured to pressurize the medicinal liquid stored in the chamber 100. The piston 200 may be configured to be slidable to pressurize the medicinal liquid stored in the chamber 100. The piston 200 is movable while maintaining an airtight state with the inner surface of the chamber housing 101. The piston 200 is capable of partitioning a medicinal liquid storage space 105 filled with medicinal liquid flowing in through the medicinal liquid inflow part 110, and a pressurizing space 205. The gas generated from the pressurizing actuator 300 can be supplied to increase a pressure in the pressurizing space 205.

The piston 200 includes a pressuring surface facing the medicinal liquid storage space 105 of the chamber 100. The pressurizing surface may come into contact the medicinal liquid inside the chamber 100 and directly push the medicinal liquid. The piston 200 having the pressurizing surface can pressurize the medicinal liquid inside the chamber 100 by moving in a predetermined pressurizing direction Ap1. Here, the pressurizing direction Ap1 may be a direction approaching the medicinal liquid injection part 120.

When the medicinal liquid is being filled into the medicinal liquid storage space 105 inside the chamber 100, the pressurizing surface moves in the direction Ap2 opposite to the pressurizing direction Ap 1. FIGS. 2 and 3 illustrate the piston 200' when the piston moves a predetermined distance in the pressurizing direction Ap 1. However, the position of the piston 200 is not limited by the position illustrated in the drawing.

The piston 200 may include a first part 210 having the pressurizing surface and a second part 220 coupled to the first part 210. The second part 220 may be coupled to the first part 210 at the lower side of the first part 210. The second part 220 is configured to move integrally with the first part 210.

The pressurizing actuator 300 may be configured to provide power such that the piston 200 moves in the pressurizing direction Ap1. As an example (not illustrated), the pressurizing actuator 300 may provide a portion that can be held by a user, so that the piston 200 can be moved in the pressurizing direction Ap1 by the user's force. As another example (not illustrated), the pressurizing actuator 300 may be configured to pressurize the liquid by using the elastic force of an elastic body such as a balloon. In this case, the pressurizing actuator 300 may be configured to pressurize the liquid in the balloon.

Hereinafter, the pressurizing actuator 300 will be described based on an embodiment in which the gas is generated, but the present disclosure is not limited thereto.

In an embodiment, the pressurizing actuator 300 may be configured to increase the pressure of gas in the pressurizing space 205 formed in a direction opposite to the pressurizing direction Ap1 of the piston 200. The pressurizing actuator 300 may include a receptacle 310 configured to accommodate a liquid substance 7 and a solid substance 6 that generates gas when coming into contact with the liquid substance 7. The pressurizing actuator 300 may include a partition 320 that isolates the liquid substance 7 and the solid substance 6 from each other.

Referring to FIGS. 2 to 4, the receptacle 310 may include an extension 313 extending from the inside to the pressurizing space 205. The extension 313 forms a hollow portion connected to the interior of the receptacle 310, and the hollow portion is closed by a stopper not to be connected to the pressurizing space 205. The extension 313 may have a substantially cylindrical shape. When manufacturing the receptacle 310, the liquid substance 7 may move into the receptacle 310 through the extension 313. After the liquid substance 7 passes through the extension 313 and is received inside the receptacle 310, the extension 313 can be closed with the stopper. The movement of the liquid substance 7 through the extension 313 can be blocked by the stopper.

The receptacle 310 may include a boss 314 extending into the pressurizing space 205. The boss 314 may be provided with a pressure regulating valve 315 configured to prevent the pressure of the gas from exceeding a preset pressure. In order to prevent the medicinal liquid from being injected into the patient faster than a predetermined flow rate, when the pressure in the pressurizing space 205 becomes higher than the preset pressure, the pressure regulating valve 315 connects the pressurizing space 205 to the outside (e.g., the air) such that the gas in the pressurizing space 205 is discharged to the outside. As a result, the pressure of the pressurizing space 205 can be maintained constant.

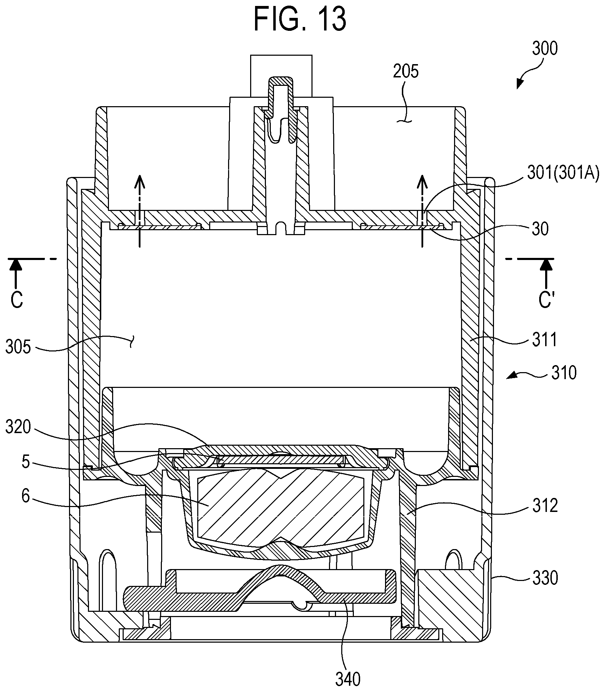

The pressurizing actuator 300 may be provided with an air vent hole 301 configured to allow gas moving from the inside of the receptacle 310 to the pressurizing space 205 to pass therethrough. The medicinal liquid injection apparatus 10 may include a pressing-part filter 30 arranged to allow gas moving into the pressurizing space 205 through the air vent hole 301 to pass therethrough. The pressing-part filter 30 may be configured to be impermeable to liquid and permeable to gas. The pressing-part filter 30 may be hydrophobic. The pressing-part filter 30 will be described later with reference to FIGS. 13 to 16.

The receptacle 310 may have a liquid accommodation portion 305 configured to accommodate the liquid substance 7. The receptacle 310 may have an opening 307 that is open in the direction facing the liquid accommodation portion 305. The receptacle 310 may include a solid accommodation portion 306 configured to accommodate the solid substance 6. The receptacle 310 may include an upper receptacle housing 311 and a lower receptacle housing 312. The liquid accommodation portion 305 may be formed inside the upper receptacle housing 311. The solid accommodation portion 306 may be formed inside the lower receptacle housing 312.

The liquid accommodation portion 305 and the solid accommodation portion 306 of the receptacle 310 may be separated by a partition 320. The partition 320 may isolate the liquid substance 7 and the solid substance 6 from each other by closing the opening 307 at a closed position. The closed position may be a position where the partition 320 is coupled to the receptacle 310 to isolate the liquid substance 7 and the solid substance 6 from each other, as illustrated in FIGS. 2 and 3.

The partition 320 may be coupled to the receptacle 310 with an adhesive. The partition 320 may be coupled to the lower receptacle housing 312. Wax may be applied to the lower receptacle housing 321. The partition 320 may be bonded to a surface of the lower receptacle housing 321 to which the wax is applied. The partition 320 may be bonded to the lower receptacle housing 312 by using adhesive or ultraviolet (UV) adhesive.

The partition 320 may be configured to break away from the closed position when an external force is applied to the receptacle 310. When an external force is applied to the receptacle 310, the coupling of the partition 320 to the receptacle 310 may be released, and the partition 320 may be separated from the lower receptacle housing 312.

As an example, the lower receptacle housing 312 of the receptacle 310 may be made of a flexible material. The lower receptacle housing 312 made of the flexible material is elastically deformable by being pressed by an external force. When the lower receptacle housing 312 is elastically deformed to press the solid substance 6, the solid substance 6 may press the partition 320. Accordingly, the partition 320 may move away from the closed position.

When the partition 320 moves away from the closed position, the solid substance 6 may escape into the liquid accommodation portion 305, and the liquid substance 7 and the solid substance 6 may come into contact with each other. When the liquid substance 7 and the solid substance 6 come into contact with each other, gas (e.g., carbon dioxide) may be generated. The gas generated within the receptacle 310 may move to the pressurizing space 205 through the air vent hole 301, and thus the pressure of air within the pressurizing space 205 may increase. As the air pressure in the pressurizing space 205 increases, the piston 200 moves in the pressurizing direction Ap 1.

The solid accommodation portion 306 may accommodate a boosting substance 5 together with the solid substance 6. The boosting substance 5 may come into contact with the liquid substance 7 before the solid substance 6 at the moment the partition 320 moves away from the closed position, thereby rapidly increasing the amount of gas generated at the beginning of the reaction. Accordingly, even at the beginning of the reaction when the reaction between the solid substance 6 and the liquid substance 7 is not sufficient, the pressure of the gas in the pressurizing space 205 can be sufficiently increased.

The solid substance 6 may be, for example, in the form of pellets containing sodium bicarbonate as a main ingredient. The liquid substance 7 may be an acidic liquid substance 7 such as citric acid that generates carbon dioxide when reacting with the solid substance 6. The boosting substance 5 may contain a high concentration of solid substance 6, that is, a high concentration of sodium bicarbonate as its main ingredient.

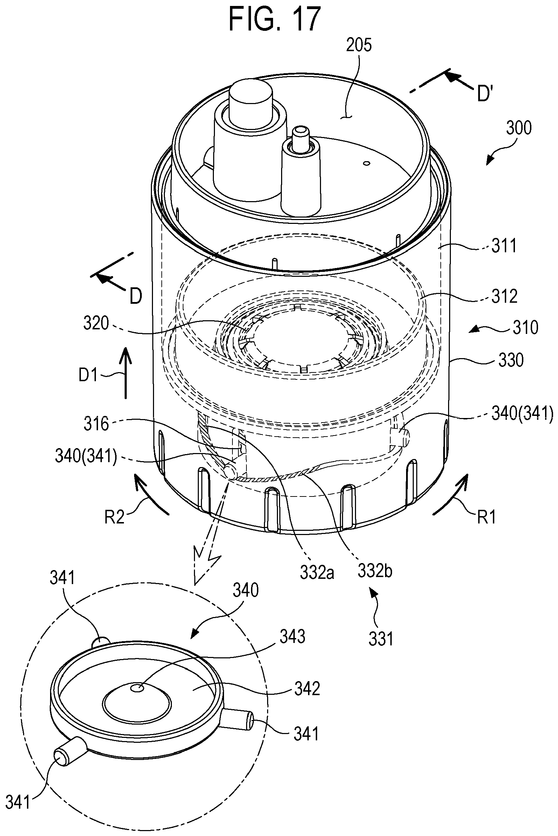

Meanwhile, the pressurizing actuator 300 may include a rotary part 330 rotatably coupled to the receptacle 310. The pressurizing actuator 300 may include a pressing part 340 configured to move in a direction approaching the receptacle 310 as the rotary part 330 rotates. The configurations of the rotary part 330 and the pressing part 340 will be described later with reference to FIGS. 17 to 20.

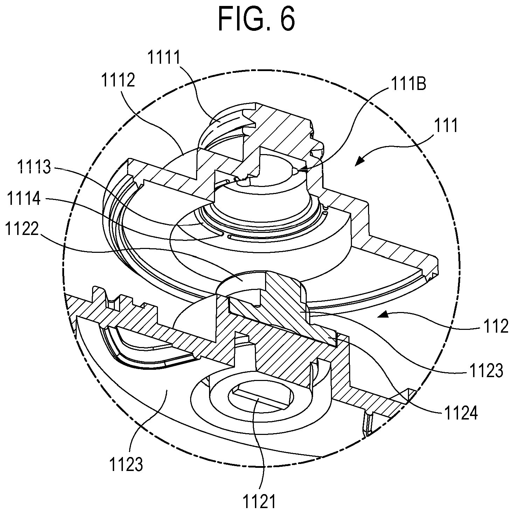

FIG. 5 is an enlarged view of a medicinal liquid inflow part 110 in the medicinal liquid injection apparatus 10 illustrated in FIG. 4. FIG. 6 is a cross-sectional perspective view illustrating the medicinal liquid inflow part 110 of FIG. 5 partially sectioned in the state in which the cap 111 and the base 113 are separated.

Referring to FIGS. 5 and 6, the packing 112 may include a first pillar 1122. The packing 112 may include a second pillar 1123 connected to the lower end of the first pillar 1122. The second pillar 1123 may have a larger outer diameter than the outer diameter of the first pillar 1122.

The packing 112 may include a pinched portion 1124 extending outward. The pinched portion 1124 may extend outward from the lower end of the second pillar 1123. The pinched portion 1124 may be configured to be fixedly interposed between the cap 111 and the base 113.

The packing 112 may include a medicinal liquid inflow hole 1121. The packing 112 may regulate the flow of the medicinal liquid by using the medicinal liquid inflow hole 1121. The packing 112 may be configured such that the medicinal liquid inflow hole 1121 is open to allow the flow of the medicinal liquid from the outside into the inside of the chamber 100 and is closed to prevent the flow of the medicinal liquid from the inside of the chamber 100 to the outside. Accordingly, the medicinal liquid inflow hole 1121 can prevent the medicinal liquid from flowing back from the inside of the chamber 100 to the outside. The medicinal liquid inflow hole 1121 may function as, for example, a check valve (one-way valve).

In an embodiment, the packing 112 may include protruding portions 1125 that protrude in the direction of the inflow flow of the medicinal liquid. The medicinal liquid inflow hole 1121 is formed at the protruding ends of the protruding portions 1125. The hole 751a is formed to allow a flushing liquid to pass therethrough. The medicinal liquid inflow hole 1121 is open or closed depending on the flow direction of the medicinal liquid. When the medicinal liquid flows from the outside into the inside of the chamber 100, the protruding portions 1125 spread to both sides to open the medicinal liquid inflow hole 1121, and when the medicinal liquid flows from the inside to the outside of the chamber 100, the protruding portions 1125 retract to close the medicinal liquid inflow hole 1121.

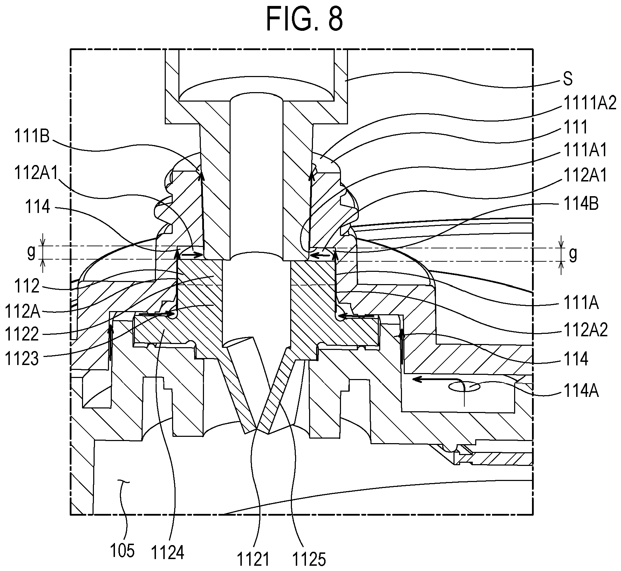

The cap 111 may include a first cylinder 1111. The cap 111 may include a second cylinder 1112 having an inner diameter larger than the inner diameter of the first cylinder 1111. The cap 111 defines a through hole extending through the center of the cap 111. The through hole may extend through the first cylinder 1111 and the second cylinder 1112. A medicinal liquid introduction tool S (see FIG. 8) such as a syringe may be inserted into the through hole. At least one recessed portion 111B may be formed in the first cylinder 1111. As illustrated in FIG. 6, the cap 111 may include a compression portion 1113 that protrudes in a direction toward the pinched portion 1124 of the packing 112. The compression portion 1113 may come into contact with the pinched portion 1124 to fix the pinched portion 1124. At least one opening 1114 may be formed in the compression portion 1113. The compression portion 1113 can fix the packing 112 at a predetermined position between the cap 111 and the base 113 by pressing the pinched portion 1124 of the packing 112. The at least one opening 1114 formed in the compression portion 1113 may be configured to allow air to pass therethrough. For example, the at least one opening 1114 may be formed along the outer periphery of the compression portion 1113, and air may pass through the opening 1114 to be discharged to the outside.

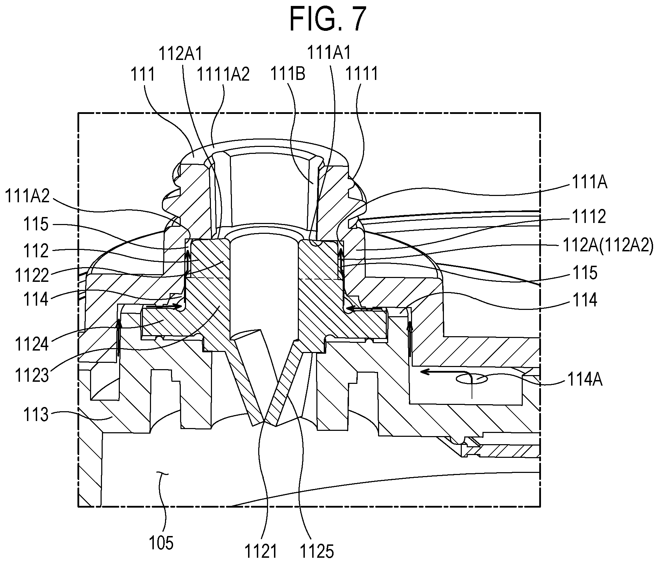

FIG. 7 is an enlarged view of a portion including the medicinal liquid inflow part 110 in the medicinal liquid injection apparatus 10 illustrated in FIG. 3.

Referring to FIG. 7, the inner surface 111A of the cap 111 may define an air exhaust hole 114 that allows the medicinal liquid storage space 105 inside the chamber 100 to fluidly communicate with the outside. The air exhaust hole 114 may be a passage space formed between the cap 111 and the packing 112 and/or between the cap 111 and the base 113 to allow air to flow therethrough. The air exhaust hole 114 includes an inlet 114A of the air exhaust hole 114 extending into the medicinal liquid storage space 105. The inlet 114A of the air exhaust hole 114 may be formed in the bottom surface of the base 113 and extend to the top surface of the base 113.

The outer surface 112A of the packing 112 may be configured to close the air exhaust hole 114 by being brought into contact with the inner surface 111A of the cap 111. The top surface 112A1 of the packing 112 is in airtight contact with the bottom surface 111A1 of the first cylinder 1111 of the cap 111 that partitions a portion of the air exhaust hole 114, thereby closing the air exhaust hole 114. As the air exhaust hole 114 is closed by the packing 112, fluid communication through the air exhaust hole 114 is impossible. As illustrated in FIG. 7, when the packing 112 is not deformed since no external force is applied to the packing 112, fluid communication through the air exhaust hole 114 is impossible since the gas in the medicinal liquid storage space 105 cannot pass through the air exhaust hole 114.

The outer peripheral surface 112A2 of the packing 112 and the inner peripheral surface 111A2 of the cap 111 may face each other. A predetermined separation space 115 may be formed between the outer peripheral surface 112A2 of the packing 112 and the inner peripheral surface 111A2 of the cap 111. The separation space 115 may be formed between the outer peripheral surface 112A2 of the first pillar 1122 of the packing 112 and the inner peripheral surface 111A2 of the second cylinder 1112 that partitions a portion of the air exhaust hole 114.

FIG. 8 is a view illustrating a portion including the medicinal liquid inflow part 110 in FIG. 7 when the packing 112 is pressed from the outside and elastically deformed.

Referring to FIG. 8, when the packing 112 is pressed from the outside and elastically deformed, a gap g capable of fluid communication may be formed between the outer surface 112A of the packing 112 and the inner surface 111A of the cap 111. The gap g may be formed such that the air exhaust hole 114 is open. The packing 112 may be pressed by, for example, the medicinal liquid introduction tool S for introducing a medicinal liquid. The medicinal liquid introduction tool S may be, for example, a syringe. When the packing 112 is pressed from the outside, a portion of the packing 112 may be elastically deformed to be introduced into the separation space 115 (see FIG. 7). When the packing 112 is pressed from the outside, a part of the packing 112 may be compressed and introduced into the separation space 115, and the contact between the bottom surface 111A1 of the first cylinder 1111 and the top surface 112A1 of the packing 112 may be smoothly released to form a gap g. Since the separation space 115 into which a portion of the packing 112 can be compressed and introduced is provided, the gap g can be effectively created. As the gap g is formed, the air exhaust hole 114 may be open. As the air exhaust hole 114 is open, the air inside the medicinal liquid storage space 105 can be discharged to the outside through the air exhaust hole 114. The air inside the medicinal liquid storage space 105 may flow into the inlet 114A of the air exhaust hole 114 and then be discharged to the outside through the gap g. Accordingly, fluid communication is possible through the air exhaust hole 114.

When the packing 112 is pressed from the outside, the at least one recessed portion 111B, which is formed on the inner peripheral surface 111A2 of the first cylinder 1111 to extend from the top surface 1111A2 to the bottom surface 111A1 of the first cylinder 1111, may be connected to the gap g. At positions corresponding to a plurality of recessed portions 111B, the portions of the top surface of the packing 112, which are in contact with the bottom surface of the first cylinder 1111, may be released from contact. The plurality of recessed portions 111B may be connected to the gap g to facilitate fluid communication through the air exhaust hole 114.

When the medicinal liquid is filled according to the opening and closing of the above-described air exhaust hole 114, that is, when the packing 112 is pressed and deformed, the gas in the medicinal liquid storage space 105 can be discharged to the outside of the medicinal liquid injection apparatus 10. Therefore, the gas in the medicinal liquid storage space 105 can smoothly escape to the outside, and the medicinal liquid can be prevented from flowing out to the outside.

FIG. 9 is an elevation view of the medicinal liquid inflow portion and the hydrophobic filter illustrated in FIG. 2 when viewed in the directions of arrows B and B', and FIG. 10 is a view in which the hydrophobic filter in FIG. 9 is omitted.

Referring to FIGS. 9 and 10, the hydrophobic filter 20 may be configured to be impermeable to liquid and permeable to gas. The hydrophobic filter 20 may allow only gas to pass through the portion where the hydrophobic filter 20 is disposed. The hydrophobic filter 20 may be disposed on the bottom side of the base 113 of the medicinal liquid inflow part 110. Here, the inlet 114A of the air exhaust hole 114 may be formed on the bottom side of the base 113. The hydrophobic filter 20 may be disposed to cover the inlet 114A of the air exhaust hole 114 from the bottom side. The hydrophobic filter 20 may be disposed such that air moving from the inside to the outside of the chamber 100 (see FIG. 2) through the inlet 114A of the air exhaust hole 114 passes through the hydrophobic filter 20. The hydrophobic filter 20 may be disposed to prevent the liquid (e.g., medicinal liquid) stored inside the chamber 100 from moving to the outside through the inlet 114A of the air exhaust hole 114.

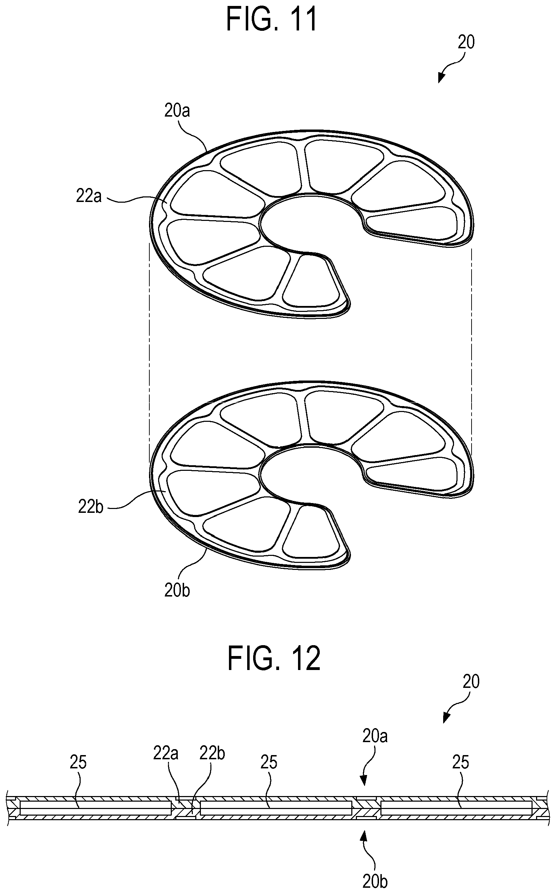

FIG. 11 is an exploded perspective view of the hydrophobic filter 20 illustrated in FIG. 9.

Referring to FIG. 11, the hydrophobic filter 20 may include a first hydrophobic filter layer 20a and a second hydrophobic filter layer 20b. The first hydrophobic filter layer 20a and the second hydrophobic filter layer 20b may be bonded to each other to form the hydrophobic filter 20.

FIG. 12 is a cross-sectional view of the hydrophobic filter 20 illustrated in FIG. 11 taken in a lateral direction perpendicular to the stacking direction of the first and second hydrophobic filter layers 20b.

Referring to FIGS. 9 to 12, the first hydrophobic filter layer 20a and the second hydrophobic filter layer 20b may be bonded to each other to define a plurality of block boundaries 21. As illustrated in FIG. 9, the plurality of block boundaries 21 may be arranged in an annular shape. The plurality of block boundaries 21 may each partition a range in which liquid is movable in a lateral direction perpendicular to the stacking direction of the first hydrophobic filter layer 20a and the second hydrophobic filter layer 20b. The plurality of block boundaries 21 may respectively define a plurality of block boundary areas 25 as ranges in which liquid is movable. The plurality of block boundaries 21 may prevent liquid from moving from one block boundary area 25 to another block boundary area 25 which is adjacent to the one block boundary area 25 in the lateral direction. Since the plurality of block boundaries 21 partition the plurality of block boundary areas 25, which are the ranges in which the liquid is movable in the lateral direction, the liquid can be prevented from passing through the hydrophobic filter 20 in units of block boundary areas 25. That is, since the flow of liquid in the lateral direction is prevented between two hydrophobic filter layers 20a and 20b, even when a portion of a first hydrophobic filter layer 20a within one block boundary area 25 partitioned by one block boundary 21 is damaged and a portion of a second hydrophobic filter layer 20b within another block boundary area 25 partitioned by another boundary block 21 is damaged, liquid can be prevented from passing through the hydrophobic filter 20. Therefore, the impact of partial damage to the hydrophobic filter 20 can be minimized and leakage of the medicinal liquid can be effectively prevented.

As an example, the first hydrophobic filter layer 20a may include a first block boundary forming portion 22a. The second hydrophobic filter layer 20b includes a second block boundary forming portion 22b. The first block boundary forming portion 22a and the second block boundary forming portion 22b may form a plurality of block boundaries 21 together. The first block boundary forming portion 22a and the second block boundary forming portion 22b may protrude from the first hydrophobic filter layer 20a and the second hydrophobic filter layer 20b, respectively, in directions facing each other.

As another example, the first hydrophobic filter layer 20a and the second hydrophobic filter layer 20b may be partially bonded to each other, so that a plurality of block boundaries 21 may be formed by the bonded portions. The bonded portions may form structures 22a and 22b interconnecting the first hydrophobic filter layer 20a and the second hydrophobic filter layer 20b in the stacking direction. The first hydrophobic filter layer 20a and the second hydrophobic filter layer 20b may be thermally bonded to each other. For example, when heat is applied after the first hydrophobic filter layer 20a and the second hydrophobic filter layer 20b are brought into contact with each other, the first hydrophobic filter layer 20a and the second hydrophobic filter layer 20b can be bonded to each other while being in close contact with each other along the structures 22a and 22b.

Referring to FIG. 9 together, the inlet 114A of the air exhaust hole 114 may be formed in the bottom surface of the base 113. The plurality of block boundary areas 25 may be formed to correspond to the inlet 114A of the air exhaust hole 114. The inlet 114A of the air exhaust hole 114 is formed at a location corresponding to the block boundary areas 25 partitioned by the plurality of block boundaries 21.

As an example, the inlet 114A of the air exhaust hole 114 may be formed to extend in the bottom surface of the base 113 in an annular shape to correspond to the plurality of block boundary areas 25.

As another example (not illustrated), the inlet 114A of the air exhaust hole 114 may be formed in plural numbers. The plurality of air exhaust holes 114 may be formed to be spaced apart from each other along the circumference. The plurality of block boundary areas 25 of the hydrophobic filter 20 may be arranged to correspond to respective inlets 114A of the air exhaust hole 114 formed to be spaced apart from each other.

The air inside the chamber 100 needs to escape to the outside through the air exhaust hole 114. However, liquid may also flow through the formed air exhaust hole 114. In this case, the liquid stored inside the chamber 100, for example, a medicinal liquid, may leak. In the hydrophobic filter 20 according to the present disclosure, the plurality of block boundaries 21 are formed so that liquid penetration can be prevented in units of block boundaries 21.

FIG. 13 is an enlarged view illustrating the pressurizing actuator 300 in the medicinal liquid injection apparatus 10 illustrated in FIG. 2.

Referring to FIG. 13, in the pressurizing actuator 300, air vent holes 301 may be formed to guide gas generated within the receptacle 310 to the pressurizing space 205. The air vent holes 301 may be predetermined passage spaces that allow the gas generated by the reaction between the solid substance 6 and the liquid substance 7 accommodated in the receptacle 310 to flow therethrough. For example, carbon dioxide generated within the receptacle 310 may move into the pressurizing space 205 through the air vent holes 301. The gas pressure in the pressurization space 205 increases due to the gas moving into the pressurization space 205, and the piston 200 can move in the pressurization direction Ap1 (see FIG. 2). The medicinal liquid injection apparatus 10 may include a pressing-part filter 30. The pressing-part filter 30 may be arranged to allow gas moving from the inside of the receptacle 310 to the pressurizing space 205 through the air vent holes 301 to pass therethrough.

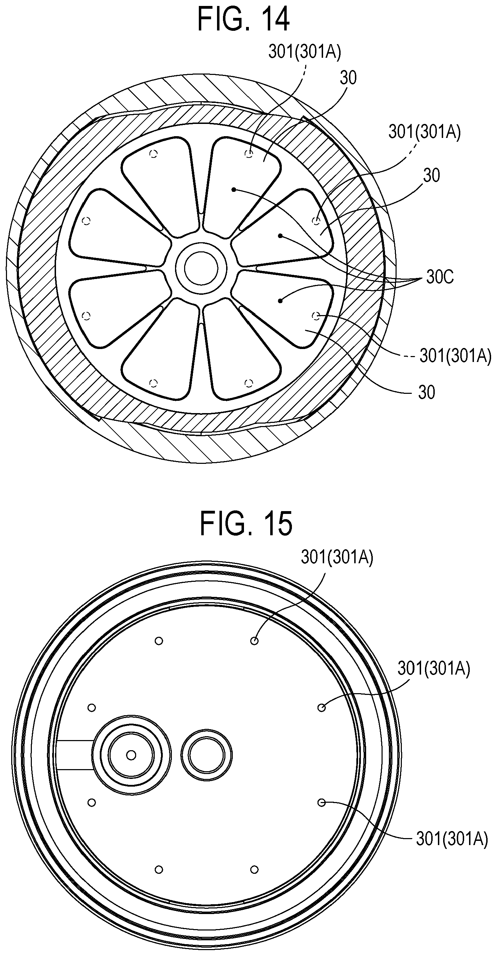

FIG. 14 is a cross-sectional view of the pressurizing actuator 300 taken along C-C' in FIG. 13, and FIG. 15 is a view in which a plurality of pressing-part filters 30 are omitted from FIG. 14.

Referring to FIGS. 13 to 15, the air vent hole 301 may include inlets 301A of a plurality of air vent holes 301. Carbon dioxide generated within the receptacle 310 may flow, through the inlets 301A of the plurality of air vent holes 301, into the air vent holes 301 and then move to the pressurizing space 205.

The plurality of pressing-part filters 30 may be configured to be impermeable to liquid and permeable to gas. The pressing-part filters 30 may allow only gas to pass through the portions where the hydrophobic filters 30 are disposed. The plurality of pressing-part filters 30 may be arranged to be spaced apart from each other and cover the inlets 301A of the plurality of air vent holes 301, respectively. The plurality of pressing-part filters 30 may be disposed at positions corresponding to respective inlets 301A of the plurality of air vent holes 301. The pressing-part filters 30 may be arranged such that the gas moving from the inside of the receptacle 310 to the pressurizing space 205 passes through the pressing-part filters 30, and the liquid stored inside the receptacle 310 cannot move to the outside through the air vent holes 301.

As an example, as illustrated in FIG. 14, the inlets 301A of the air vent holes 301 may be formed in the circumferential direction around an axis parallel to the pressurizing direction. The plurality of pressing-part filters 30 may also be arranged to be spaced apart from each other in the circumferential direction centered on the axis parallel to the pressurizing direction. Here, the inlets 301A of the air vent holes 301 may be formed at positions farther from the axis than the centers 30C of the corresponding pressing-part filters 30. The inlets 301A of the air vent holes 301 may be located closer to the upper receptacle housing 311 than the centers 30C of the corresponding pressing-part filters 30.

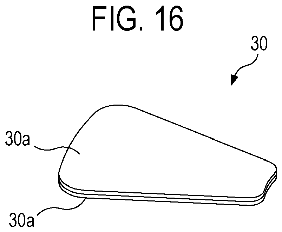

FIG. 16 is a perspective view of the pressing-part filter 30 illustrated in FIG. 14.

Referring to FIGS. 14 and 16, each of the plurality of pressing-part filters 30 may include a plurality of stacked hydrophobic filter layers. As an example, the pressing-part filter 30 may include a first pressing-part filter layer 30a. The pressing-part filter 3 may include a second pressing-part filter layer 30b. The first pressure part filter layer 30a and the second pressure part filter layer 30b may be bonded to each other. Here, the first pressing-part filter layer 30a and the second pressing-part filter layer 30b may be thermally bonded to each other. For example, when heat is applied after the first pressing-part filter layer 30a and the second pressing-part filter layer 30b are brought into contact, the first pressing-part filter layer 30a and the second pressing-part filter layer 30b can be bonded to each other while being brought into close contact with each other. Here, the edge portion of the first pressure portion filter layer 30a and the edge portion of the second pressure portion filter layer 30b may be bonded to each other.

As another example (not illustrated), the hydrophobic filter 20 described above with reference to FIGS. 9 to 12 may be disposed to allow gas moving to the pressurizing space 205 from the inside of the receptacle 310 through the vent holes 301 to pass therethrough. The plurality of block boundaries 21 of the hydrophobic filter 20 partition a plurality of block boundary areas 25, and the hydrophobic filter 20 may be disposed such that the plurality of block boundary areas 25 cover the plurality of air vent holes 301, respectively. The inlets 301A of the plurality of air vent holes 301 may be formed at positions corresponding to the plurality of block boundary areas 25 partitioned by the plurality of block boundaries.

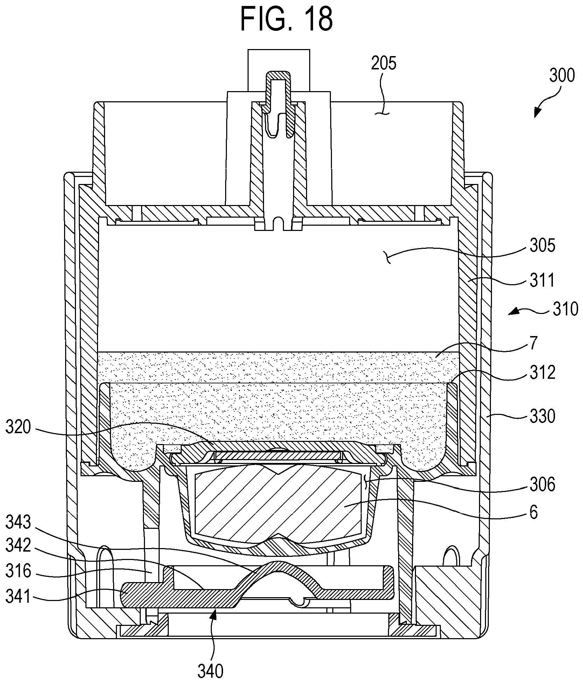

FIG. 17 is a projection view of one aspect of the pressurizing actuator 300 illustrated in FIG. 13, and FIG. 18 is a cross-sectional view of the pressurizing actuator 300 taken along line D-D' in FIG. 17.

Referring to FIGS. 17 and 18, the rotary part 330 of the pressurizing actuator 300 may be configured to rotate with respect to a receptacle 310. For example, the rotary part 330 may be rotatably coupled to the lower receptacle housing 312 of the receptacle 310. As the rotary part 330 rotates, the pressing part 340 of the pressurizing actuator 300 may be configured to be pressed and moved in an upward direction (hereinafter, referred to as a "first direction D1"), which is a direction approaching the receptacle 310. The pressing part 340 may be configured to release an isolation by the partition 320 by pressing the receptacle 310 by moving in the first direction D1. The pressing part 340 may cause the partition 320 to move away from the closed position by pressing the receptable 310.

The rotary part 330 may rotate with respect to the receptacle 310 with an axis in an up and down direction, that is, an axis parallel to the first direction D1, as a rotation axis. As an example, the rotary part 330 is rotatable in a first rotation direction R1 illustrated in FIG. 17 and a second rotation direction R2 opposite to the first rotation direction R1 with an axis (virtual axis) parallel to the first direction D1 as the rotation axis. Here, the first rotation direction R1 and the second rotation direction R2 are directions presented for convenience of explanation of the rotation direction of the rotary part 330.

When a user needs to use the medicinal liquid injection apparatus 1 (see FIG. 1), the user may rotate the rotary part 330 with respect to the receptacle 310 while holding a portion other than the rotary part 330. The user may rotate the rotary part 330 in the first rotation direction R1 or the second rotation direction R2. As a result, the rotary part 330 may rotate with respect to the receptacle 310, and the pressing part 340 may move in the first direction D1 in response to the rotation of the rotary part 330.

The pressing part 340 may include a plate portion 342 disposed inside the rotary part 330 and configured to press the receptacle 310. The plate portion 342 may have a substantially disk-like shape. The pressing part 340 may include a convex portion 343 that protrudes convexly in the first direction D1 and is configured to press a predetermined portion of the receptacle 310. The convex portion 343 may protrude convexly from the plate portion 342 in the first direction D1, that is, in the upward direction approaching the receptacle 310. The convex portion 343 may press the receptacle 310 when the pressing part 340 approaches the receptacle 310. In the present embodiment, the pressing part 340 is configured to press the receptacle 310 by moving from a state spaced apart from the receptacle 310 in the direction approaching the receptacle 310. However, in another embodiment (not illustrated), the pressing part 340 may be configured to press the receptacle 310 by moving from a state already in contact with the receptacle 310 in a direction approaching the receptacle 310. The pressure applied to the receptacle 310 can be concentrated to a predetermined portion by the convex portion 343.

The pressing part 340 may include at least one protrusion 341 that comes into contact with the rotary part 330. The protrusion 341 may extend outward from the plate portion 342. As illustrated in FIG. 17, the at least one protrusion 341 may be a plurality of protrusions 341 spaced apart from each other along the outer circumference of the plate portion 342. The plurality of protrusions 341 may have the same height with respect to the plate portion 342 and may be spaced apart from each other at equal intervals. In the present embodiment, three protrusions 341 are provided, but the number of protrusions 341 is not limited thereto.

The receptacle 310 may include insertion grooves 316 into which the protrusions 341 are inserted. The insertion grooves 316 may be provided in the lower receptacle housing 312 of the receptacle 310. The protrusions 341 extending outward from the plate portion 342 may pass through the insertion grooves 316 provided in the lower receptacle housing 312 from the inside to the outside. The insertion grooves 316 may extend in the first direction D 1. The insertion grooves 316 extending in the first direction D1 may guide the movement of the protrusions 341 in the first direction D 1. The insertion grooves 316 may limit the movement in the horizontal direction across the first direction D1. The horizontal width of the insertion grooves 316 may have a size corresponding to the horizontal width of the protrusions 341 to allow the protrusions 341 to be inserted into the insertion grooves while preventing the protrusions 341 from moving in the horizontal direction.

The rotary part 330 includes at least one guide portion 331. The at least one guide portion 331 may be a plurality of guide portions. The at least one guide portion 331 may be formed along the inner surface of the rotary part 330. The at least one guide portion 331 may come into contact with the pressing part 340. The at least one guide portion 331 may come into contact with the protrusions 341. The at least one guide portion 331 may include a first inclined surface 332a, the height of which in the first direction D1 is lowered along the first rotation direction R1 of the rotary part 330. The height of the first inclined surface 332a in the first direction D1 may increase along a second rotation direction R2 opposite to the first rotation direction R1.

In addition, the at least one guide portion 331 may include a second inclined surface 332b, the height of which in the first direction D1 is lowered along the second rotation direction R2 opposite to the first rotation direction R1. The height of the second inclined surface 332b in the first direction D1 may increase along the first rotation direction R1.

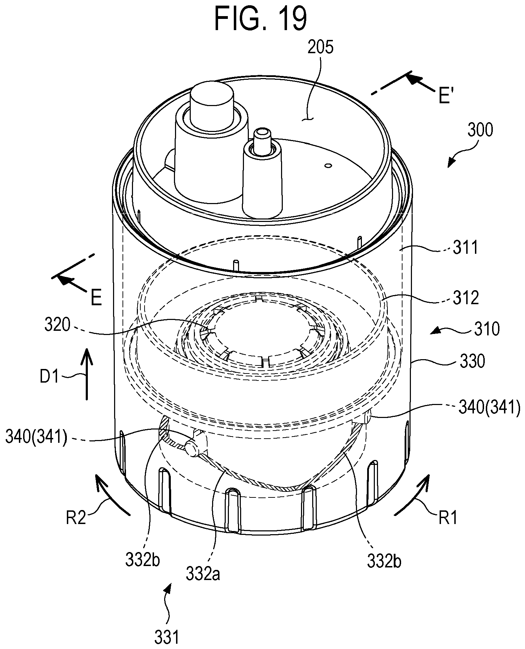

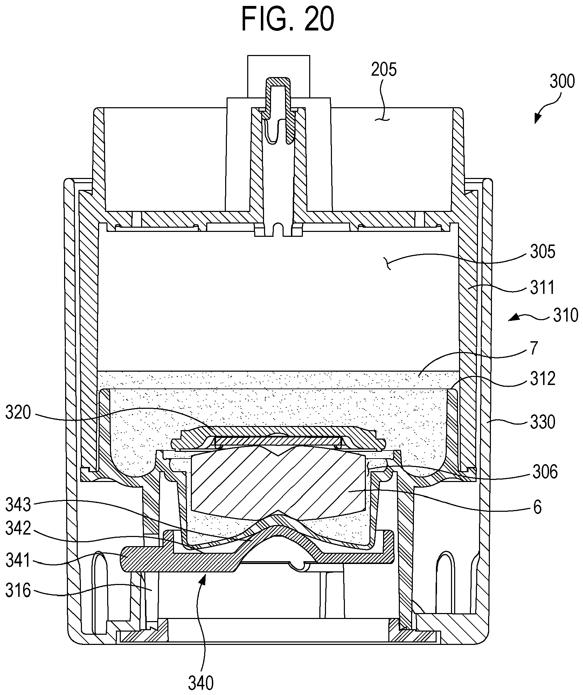

In the pre-actuation state illustrated in FIGS. 17 and 18, when a user rotates the rotary part 330 in the first rotation direction R1, the protrusion 341 of the pressing part 340 can be slid along the first inclined surface 332a. In the pre-actuation state, when a user rotates the rotary part 330 in the second rotation direction R2, the protrusions 341 of the pressing part 340 can be slid along the second inclined surface 332b. Another aspect of the pressurizing actuator 300 when a user rotates the rotary part 330 in the first rotation direction R1 or the second rotation direction R2 is illustrated in FIGS. 19 and 20.

FIG. 19 is a perspective view of the pressurizing actuator 300 illustrated in FIG. 17 in another aspect, and FIG. 20 is a cross-sectional view of the pressurizing actuator 300 taken along line E-E' in FIG. 19.

Referring to FIGS. 19 and 20 , in response to the rotation of the rotary part 330 in the first rotation direction R1, at least one guide portion 331 also rotates in the first rotation direction R1. When the at least one guide portion 331 rotates along the first rotation direction R1, the first inclined surface 332a also rotates in the first rotation direction R1. When the first inclined surface 332a rotates in the first rotation direction R1, the height of the first inclined surface 332a along which the pressing part 340 slides increases in the first direction D1. As a result, the pressing part 340 may move in the first direction D1 to press the receptacle 310. The pressing part 340 may be configured to move in the first direction D1 by sliding along the first inclined surface 332a of the at least one guide portion 331. Here, the protrusion 341 of the pressing part 340 may slide along the first inclined surface 332a of the at least one guide portion 331. The protrusion 341 is slidable along the first inclined surface 332a while being inserted into the insertion groove 316. As the insertion groove 316 limits movement in the horizontal direction, the protrusion 341 is movable only in the first direction D1 along the first inclined surface 332a, rather than rotating in the first rotation direction R1. In response to the movement of the protrusion 341 in the first direction D1, the plate portion 342 and the convex portion 343 are also movable in the first direction D 1.

In response to the rotation of the rotary part 330 in the second rotation direction R2, at least one guide portion 331 also rotates in the second rotation direction R2. When the at least one guide portion 331 rotates along the second rotation direction R2, the second inclined surface 332b also rotates in the second rotation direction R2. When the second inclined surface 332b rotates in the second rotation direction R2, the height of the second inclined surface 332b along which the pressing part 340 slides increases in the first direction D1. As a result, the pressing part 340 may move in the first direction D1 to press the receptacle 310. The pressing part 340 may be configured to move in the first direction D1 by sliding along the second inclined surface 332b of the at least one guide portion 331. Substantially the same as described above, the protrusion 341 is slidable along the second inclined surface 332b while being inserted into the insertion groove 316. At this time, the protrusion 341 is movable only in the first direction D1, rather than being rotatable in the second rotation direction R2.

The rotary part 330 may be configured to move the pressing part 340 in the first direction D1 both when rotating in the first rotation direction R1 and when rotating in the second rotation direction R2. Accordingly, regardless of the direction in which a user rotates the rotary part 330, the pressing part 340 is movable in the first direction D1. That is, a user may rotate the rotary part 330 in any one direction without distinguishing between the first rotation direction R1 and the second rotation direction R2, and as a result, the pressing part 340 may become closer to the receptacle 310. Here, a predetermined portion of the receptacle 310 can be pressed by the convex portion 343 of the pressing part 340, and as the predetermined portion of the receptacle 310 is pressed by the convex portion 343, the partition 320 can be released.

The partition 320 can be more easily and reliably released by a user by rotating the rotary part 330. In addition, since the partition 320 can be released regardless of the rotation direction of the rotary part 330, convenience when a user uses the medicinal liquid injection apparatus 10 can be improved. Therefore, the medicinal liquid injection apparatus 10 can be used more efficiently.

In the foregoing, the technical idea of the present disclosure has been described with reference to some embodiments and examples illustrated in the accompanying drawings. However, it is to be understood that various substitutions, modifications, and alterations may be made without departing from the technical idea and scope of the present disclosure that can be understood by a person ordinarily skilled in the technical field to which the present disclosure pertains. In addition, such substitutions, modifications, and alterations are to be considered as falling within the scope of the appended claims.

## Claims

1. A medicinal liquid injection apparatus comprising:
a chamber configured to store a medicinal liquid in the chamber;
a piston configured to slide in the chamber in a predetermined pressurizing direction to pressurize the medicinal liquid stored in the chamber; and
a pressurizing actuator configured to increase pressure of gas in a pressurizing space formed in a direction opposite to the pressurizing direction of the piston to press the piston,
wherein the pressurizing actuator includes:
a receptacle configured to accommodate a liquid substance and a solid substance that generates gas upon coming into contact with the liquid substance;
a partition disposed between the liquid substance and the solid substance to isolate the liquid substance and the solid substance from each other;
a rotary part rotatably coupled to the receptacle; and
a pressing part configured to move by being pressed in a first direction approaching the receptacle as the rotary part rotates, and press the receptacle by moving in the first direction to release an isolation by the partition.

2. The medicinal liquid injection apparatus of claim 1, wherein a rotation axis of rotary motion of the rotary part with respect to the receptacle is parallel to the first direction.

3. The medicinal liquid injection apparatus of claim 1, wherein the rotary part includes at least one guide portion having a first inclined surface, a height of which in the first direction is lowered along a first rotation direction of the rotary part, and
wherein, when the rotary part rotates in the first rotation direction, the pressing part is configured to move in the first direction by sliding along the first inclined surface of the guide portion.

4. The medicinal liquid injection apparatus of claim 3, wherein the at least one guide portion may further include a second inclined surface, a height of which in the first direction is lowered along a second rotation direction opposite to the first rotation direction of the rotary part, and
wherein, when the rotary part rotates in the second rotation direction, the pressing part is configured to move in the first direction by sliding along the second inclined surface of the guide portion.

5. The medicinal liquid injection apparatus of claim 3, wherein the pressing part includes:
a plate portion disposed inside the rotary part and configured to press the receptacle; and
at least one protrusion configured to be in contact with the guide portion and extending outward from the plate portion.

6. The medicinal liquid injection apparatus of claim 5, wherein the at least one guide portion may include a plurality of guide portions arranged along the rotation direction of the rotary part, and
wherein the at least one protrusion includes a plurality of protrusions each arranged along a circumference of the plate portion to be in contact with each of the plurality of guide portions, respectively.

7. The medicinal liquid injection apparatus of claim 5, wherein the receptacle includes an insertion groove into which the protrusion is inserted, and
wherein the insertion groove extends in the first direction to guide movement of the protrusion in the first direction and to limit movement of the protrusion in a lateral direction transverse to the first direction.

8. The medicinal liquid injection apparatus of claim 1, wherein the pressing part includes a convex portion protruding convexly in the first direction and configured to press a predetermined portion of the receptacle.

9. The medicinal liquid injection apparatus of claim 8, wherein the receptacle includes:
a liquid accommodation portion configured to accommodate the liquid substance; and
a solid accommodation portion having an opening open in a direction facing the liquid accommodation portion and configured to accommodate the solid substance, wherein the predetermined portion is located in the solid accommodation portion, and
wherein the partition isolates the liquid substance and the solid substance from each other by closing the opening at a closed position.
